(19) Europäisches Patentamt
European Patent Office
Office européen des brevets

(11) Veröffentlichungsnummer: **0 299 315 B1**

(12) **EUROPÄISCHE PATENTSCHRIFT**

(45) Veröffentlichungstag der Patentschrift: **16.06.93**

(51) Int. Cl.[5]: **C09B 62/085**, D06P 1/382, C07D 251/50

(21) Anmeldenummer: **88110624.9**

(22) Anmeldetag: **04.07.88**

Die Akte enthält technische Angaben, die nach dem Eingang der Anmeldung eingereicht wurden und die nicht in dieser Patentschrift enthalten sind.

(54) **Reaktivfarbstoffe.**

(30) Priorität: **16.07.87 DE 3723474**
**03.10.87 DE 3733571**

(43) Veröffentlichungstag der Anmeldung:
**18.01.89 Patentblatt 89/03**

(45) Bekanntmachung des Hinweises auf die Patenterteilung:
**16.06.93 Patentblatt 93/24**

(84) Benannte Vertragsstaaten:
**CH DE FR GB LI**

(56) Entgegenhaltungen:
**EP-A- 0 014 844** **EP-A- 0 073 178**
**EP-A- 0 131 545** **EP-A- 0 227 983**
**FR-A- 2 358 450** **FR-A- 2 384 001**

(73) Patentinhaber: **BAYER AG**

**W-5090 Leverkusen 1 Bayerwerk(DE)**

(72) Erfinder: **Stöhr, Frank-Michael, Dr.**
**Am Thelen Siefen 10**
**W-5068 Odenthal(DE)**
Erfinder: **Henk, Hermann, Dr.**
**Roggendorfstrasse 55**
**W-5000 Köln 80(DE)**
Erfinder: **Herd, Karl-Josef**
**Am Gartenfeld 66**
**W-5068 Odenthal(DE)**

EP 0 299 315 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft neue Reaktivfarbstoffe. Azoreaktivfarbstoffe mit einem substituierten Halogentriazinylrest sind bereits aus bspw. EP-A-14 844 bekannt. Zunehmende Qualitätsanforderungen erfordern aber weitere Verbesserungen.

Gegenstand der vorliegenden Erfindung sind faserreaktive Azofarbstoffe der Formel

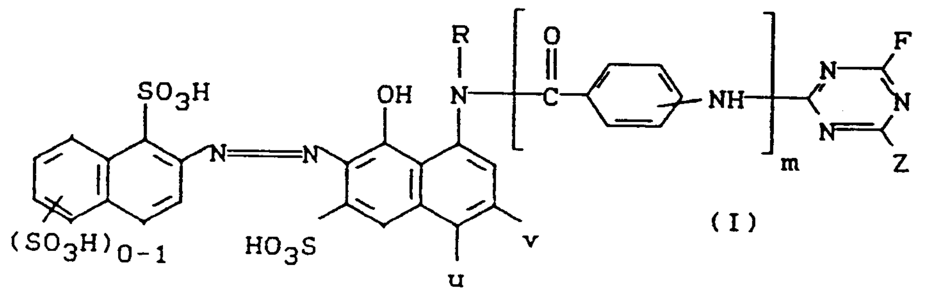

(I)

worin

u und v = H oder $SO_3H$ und $u \neq v$,

R = H oder $C_1$-$C_4$-Alkyl, wobei die Alkylgruppen gegebenenfalls durch OH, Halogen, $SO_3H$ oder $OSO_3H$ substituiert sein können,

m = 0 oder 1

Z =

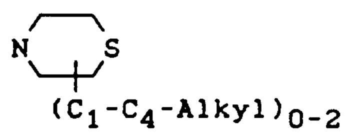 , 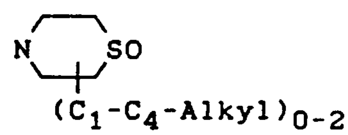

Z =

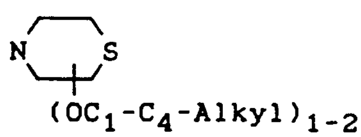 , 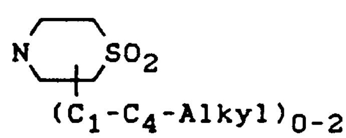

Z =

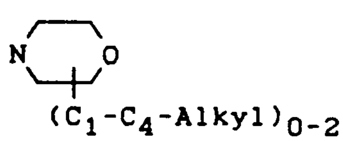 , 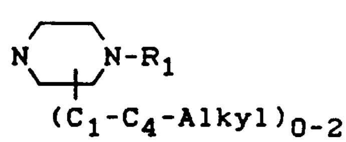

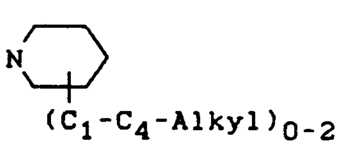 oder 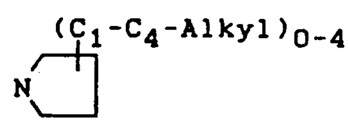

worin

$R_1$ = H oder gegebenenfalls durch wasserlöslich machende Substituenten substituiertes $C_1$-$C_6$-Alkyl.

Geeignete Substituenten für $R_1$ sind insbesondere OH, $OSO_3H$, $SO_3H$, COOH. Bevorzugter Substituent $R_1$ ist Hydroxyethyl.

Bevorzugte Farbstoffe sind solche mit Z =

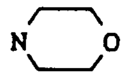 und 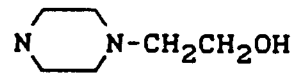

sowie solche auf Basis von 2-Aminonaphthalin-1-sulfonsäure, 2-Aminonaphthalin-1,5-disulfonsäure und 2-Aminonaphthalin-1,6-disulfonsäure. Besonders bevorzugt sind die Farbstoffe mit m = 0. Ganz besonders bevorzugt sind die Farbstoffe der Formeln

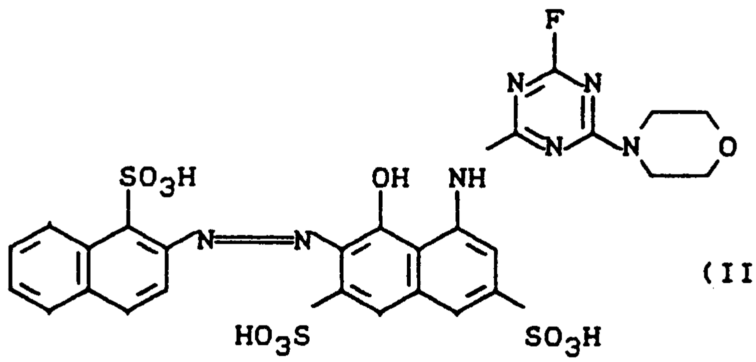

(II)

und

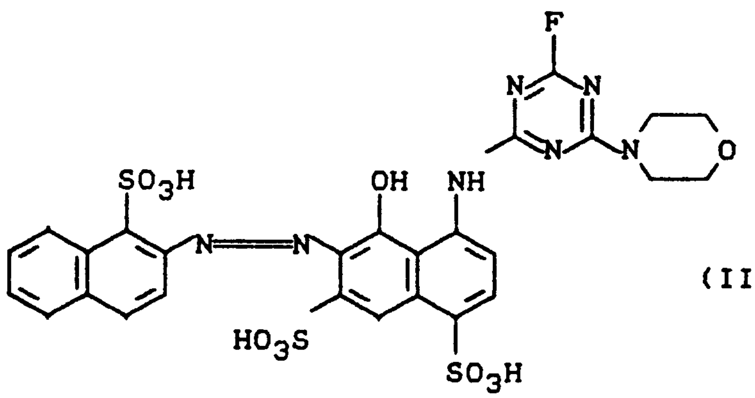

(III)

Die Farbstoffe eignen sich zum Färben und Bedrucken von hydroxyl- und amidgruppenhaltigen Materialien, insbesondere Cellulosematerialien.

Die neuen faserreaktiven Azofarbstoffe zeichnen sich durch eine hohe Reaktivität und einen hohen Fixiergrad aus.

Die mit diesen Farbstoffen erhältlichen Färbungen oder Drucke auf Cellulosematerialien zeichnen sich durch eine hohe Faser-Farbstoff-Bindungsstabilität sowie durch eine hervorragende Stabilität gegenüber Oxydationsmitteln wie peroxyd- oder chlorhaltige Waschmittel aus. Die Auswaschbarkeit der beim Färben oder Druck entstehenden Hydrolyseprodukte ist ausgezeichnet.

Die erfindungsgemäßen Farbstoffe sind nach den bei der Synthese von Reaktivfarbstoffen üblichen Herstellungsverfahren zugänglich.

So können beispielsweise die neuen Farbstoffe folgendermaßen hergestellt werden:

1. Stufe: Kondensation der Aminonaphtholdisulfonsäure mit Trifluortriazin (= Reaktionsprodukt A)

2. Stufe: Kondensation der Difluortriazinylaminonaphtholdisulfonsäure mit dem Amin H-Z (= Reaktionsprodukt B)

3. Stufe: Diazotierung der Diazokomponente und Kupplung mit Reaktionsprodukt B.

Eine weitere Möglichkeit, die Farbstoffe herzustellen, besteht darin, die Reaktionsstufen 2 und 3 zu vertauschen, d. h., man führt die Kupplung mit Reaktionsprodukt A in 2. Stufe durch und setzt erst danach

mit dem Amin H-Z in 3. Stufe um.

Ein weiteres Verfahren besteht darin, den auf üblichem Wege hergestellten Farbstoff der Formel

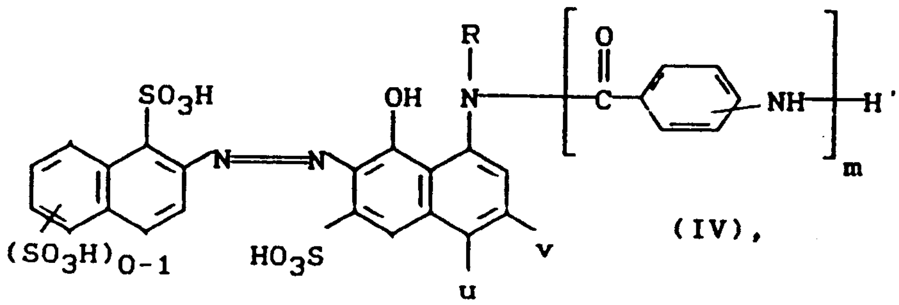

(IV),

worin

m, R, u und v die oben genannte Bedeutung haben,

mit Trifluortriazin zu kondensieren und anschließend mit dem Amin H-Z umzusetzen.

Geeignete Amine H-Z sind beispielsweise Morpholin, Thiomorpholin, Piperazin, N-Hydroxyethylpiperazin, N-Hydroxypropylpiperazin, Piperidin, Pyrrolidin, Thiomorpholin-1,1-dioxid, 2,6-Dimethylmorpholin.

Geeignete Diazokomponenten sind insbesonders 2-Aminonaphthalin-1-sulfonsäure, 2-Aminonaphthalin-1,5-disulfonsäure und 2-Aminonaphthalin-1,6-disulfonsäure.

Die angegebenen Formeln sind die der freien Säuren, Bei der Herstellung werden im allgemeinen die Salze erhalten, insbesondere die Alkalisalze wie Natrium-, Kalium- oder Lithiumsalze.

## Beispiel 1

31,9 g 1-Hydroxy-8-amino-naphthalin-3,6-disulfonsäure werden in 400 ml Wasser neutral gelöst. Bei 0-5 °C versetzt man mit 8,8 ml Trifluortriazin und hält durch Zugabe von 20 %iger Natriumcarbonatlösung einen pH-Wert von 4,0 - 4,5. Nach 5 Minuten gibt man 9 g Morpholin hinzu und hält mit 20 %iger Natriumcarbonatlösung pH 7. Nach 15 Minuten bei 10 °C ist die Reaktion beendet. Die so erhaltene Lösung der Verbindung der Formel

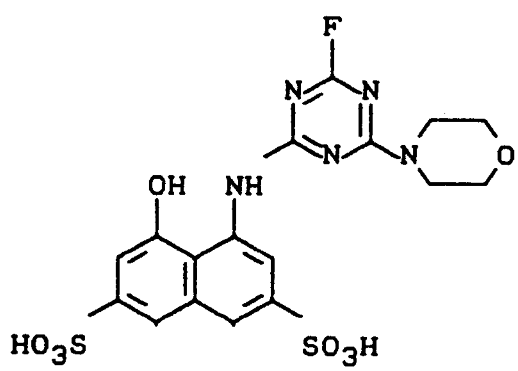

kann direkt nach der folgenden Vorschrift in einen brillanten roten Azoreaktivfarbstoff überführt werden:

Zu dem erhaltenen Reaktionsprodukt wird bei 0-5 °C eine auf üblichem Wege hergestellte Diazoniumsalz-Suspension gegeben, die durch Diazotierung von 22,3 g 2-Aminonaphthalin-1-Sulfonsäure erhalten wird. Gleichzeitig wird der pH-Wert mit 20 %iger Natriumcarbonatlösung auf 8,3 gehalten und die Kupplung zu Ende geführt. Der Farbstoff der Formel

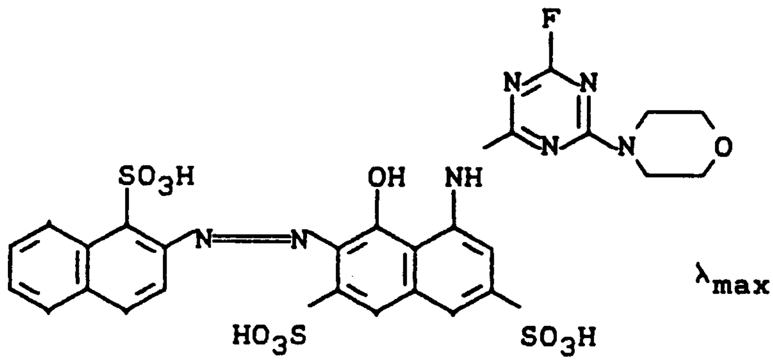

$\lambda_{max}$ = 520 nm

wird ausgesalzen, abgesaugt, getrocknet und gemahlen. Das rote Farbstoffpulver ist in Wasser leicht löslich.

Nach einem der für Reaktivfarbstoffe üblichen Applikationsverfahren erhält man damit auf Baumwolle klare blaustichig rote Färbungen.

Beispiel 2

Ersetzt man im Beispiel 1 die 1-Hydroxy-8-aminonaphthalin-3,6-disulfonsäure durch die äquivalente Menge an 1-Hydroxy-8-aminonaphthalin-3,5-disulfonsäure und arbeitet ansonsten wie im Beispiel 1 beschrieben, so erhält man den Farbstoff der Formel

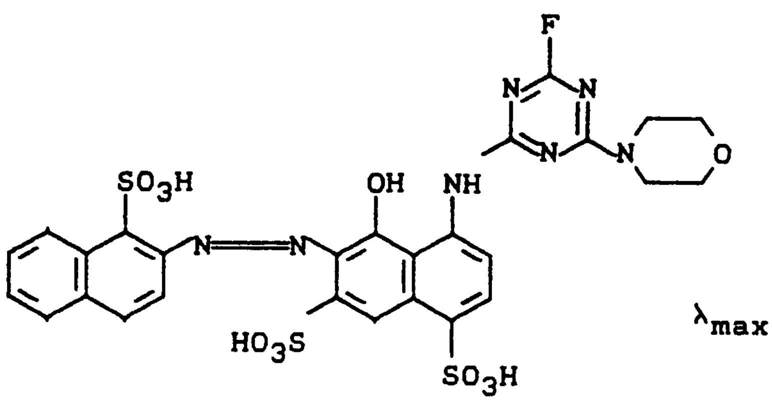

$\lambda_{max}$ = 520 nm

der ebenfalls nach einem für Reaktivfarbstoffe üblichen Applikationsverfahren Baumwolle in klaren blaustichig roten Tönen färbt.

Beispiel 3

Versetzt man die in Beispiel 1 hergestellte Lösung der Kupplungskomponente mit der nach üblichen Methoden hergestellten Diazotierung von 2-Amino-naphthalin-1,5-disulfonsäure und arbeitet ansonsten wie im Beispiel 1 angegeben, so erhält man den Farbstoff der Formel

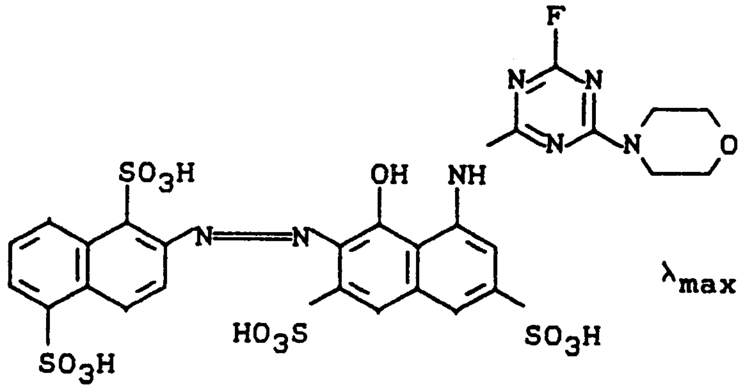

$\lambda_{max}$ = 540 nm

der ebenfalls nach einem für Reaktivfarbstoffe üblichen Applikationsverfahren Baumwolle in klaren blaustichig roten Tönen färbt.

Beispiel 4

Versetzt man die in Beispiel 2 beschriebene Kupplungskomponente mit der nach üblichen Methoden hergestellten Diazotierung von 2-Amino-naphthalin-1,5-disulfonsäure und arbeitet ansonsten wie im Beispiel 2 beschrieben, so erhält man den Farbstoff der Formel

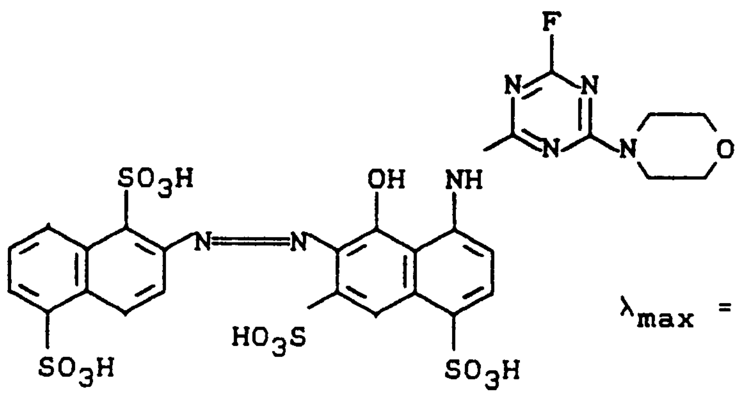

$\lambda_{max}$ = 510 nm

der ebenfalls nach einem für Reaktivfarbstoffe üblichen Applikationsverfahren Baumwolle in klaren roten Tönen färbt.

Beispiel 5

Ersetzt man in den Beispielen 1 und 2 die 2-Amino-1-naphthalinsulfonsäure durch die 2-Amino-1,6-naphthalindisulfonsäure und arbeitet ansonsten wie in den Beispielen angegeben, erhält man die Farbstoffe der Formeln

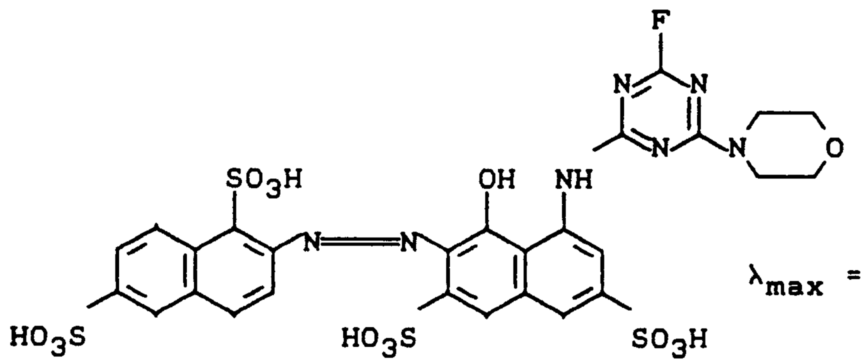

$\lambda_{max}$ = 540 nm

und

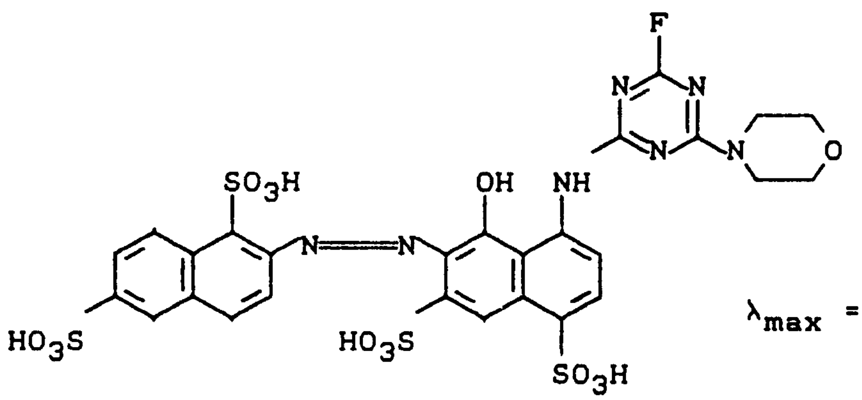

$\lambda_{max}$ = 520 nm

Diese Farbstoffe färben Baumwolle nach einem für Reaktivfarbstoffe üblichen Applikationsverfahren in klaren blaustichig roten Tönen.

Beispiel 6

Ersetzt man in den Beispielen 1-5 das bei der Herstellung der Kupplungskomponente jeweils verwendete Morpholin durch entsprechende Mengen Piperazin, N-Hydroxyethylpiperazin, Piperidin oder Pyrrolidin, so erhält man ebenfalls rote Farbstoffe mit hervorragenden Eigenschaften.

Beispiel 7

43,8 g 1-Hydroxy-8-(4-aminobenzoylamino)-naphthalin-3,6-disulfonsäure werden in 400 ml Wasser neutral gelöst. Bei 0-5 °C versetzt man mit 8,8 ml Trifluortriazin und hält durch Zugabe von 20 %iger Natriumcarbonatlösung einen pH-Wert von 4,0-4,5. Nach 5 Minuten gibt man 9 g Morpholin hinzu und stellt mit 20 %iger Natriumcarbonatlösung auf pH 7. Nach 15 Minuten bei 10 °C ist die Reaktion beendet. Die so erhaltene Lösung der Verbindung der Formel

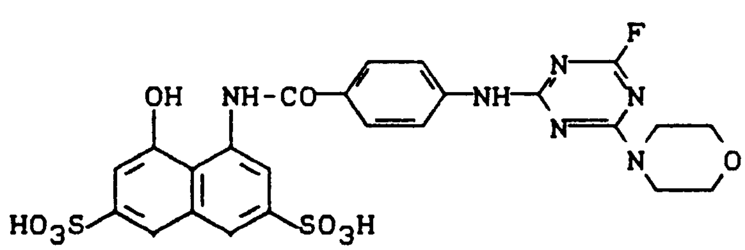

kann direkt nach der folgenden Vorschrift in einen brillanten roten Azoreaktivfarbstoff überführt werden: Zu dem erhaltenen Reaktionsprodukt wird bei 10-15 °C eine auf üblichem Wege hergestellte Diazoniumsalz-Suspension gegeben, die durch Diazotierung von 30,3 g 2-Aminonaphthalin-1,5-disulfonsäureerhalten wird. Gleichzeitig wird der pH-Wert mit 20 %iger Natriumcarbonatlösung auf 7,5 gehalten und die Kupplung zu Ende geführt. Der Farbstoff der Formel

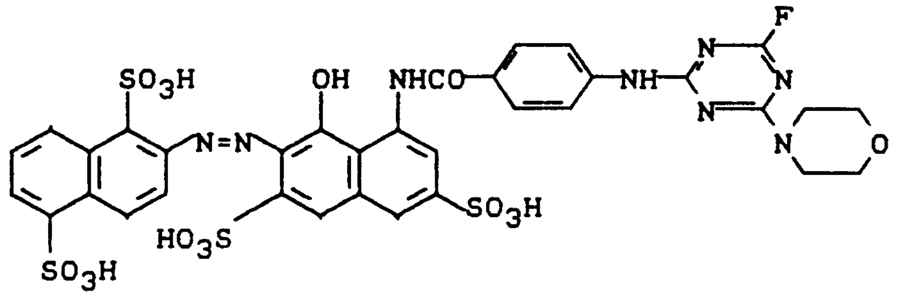

wird ausgesalzen, abgesaugt, getrocknet und gemahlen. Das rote Farbstoffpulver ist in Wasser leicht löslich. Nach einem der für Reaktivfarbstoffe üblichen Applikationsverfahren erhält man damit auf Baumwolle klare blaustichig rote Färbungen.

Beispiel 8

Ersetzt man im Beispiel 7 die 1-Hydroxy-8-(4-aminobenzoylamino)-naphthalin-3,6-disulfonsäure durch die äquivalente Menge an 1-Hydroxy-8-(4-aminobenzoylamino)-naphthalin-3,5-disulfonsäure und arbeitet ansonsten wie im Beispiel 7 beschrieben, so erhält man den Farbstoff der Formel

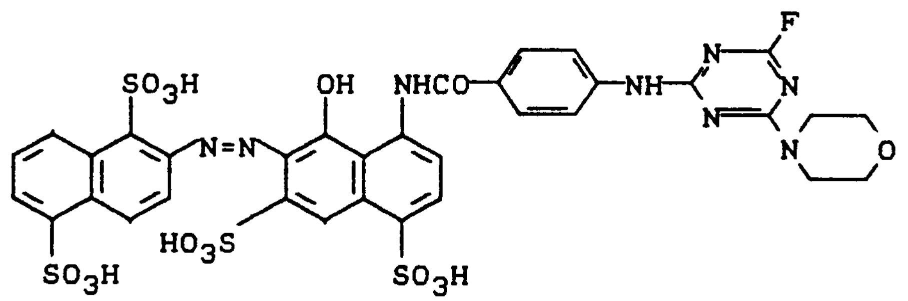

der ebenfalls nach einem für Reaktivfarbstoffe üblichen Applikationsverfahren Baumwolle in klaren blaustichig roten Tönen färbt.

Beispiel 9

Ersetzt man in den Beispielen 7 und 8 die 2-Aminonaphthalin-1,5-disulfonsäure durch die 2-Aminonaphthalin-1,6-disulfonsäure und arbeitet ansonsten wie in den Beispielen angegeben, erhält man die Farbstoffe der Formeln

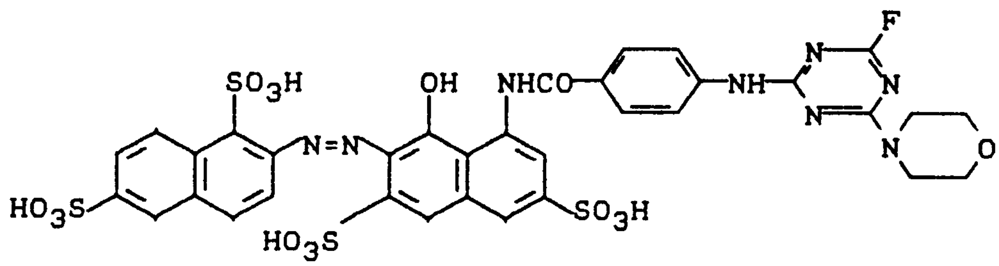

und

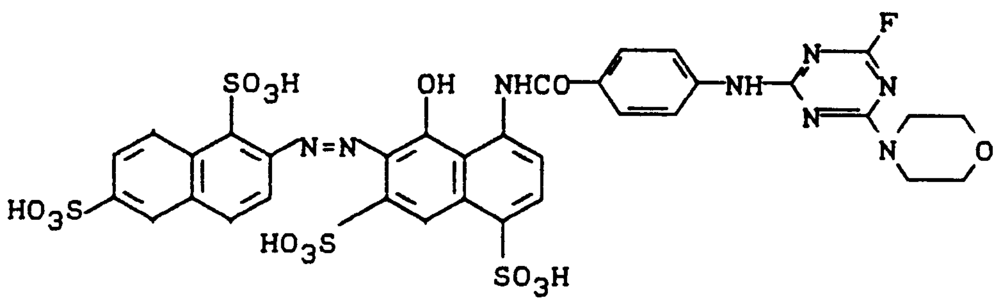

Diese Farbstoffe färben Baumwolle nach einem für Reaktivfarbstoffe üblichen Applikationsverfahren in klaren blaustichig roten Tönen.

Beispiel 10

Ersetzt man in den Beispielen 7 und 8 die 2-Aminonaphthalin-1,5-disulfonsäure durch die 2-Amino-1-naphthalinsulfonsäure und arbeitet ansonsten wie in den Beispielen beschrieben, erhält man die Farbstoffe der Formeln

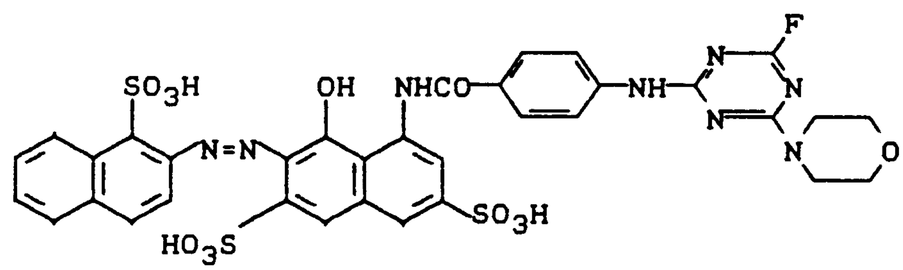

und

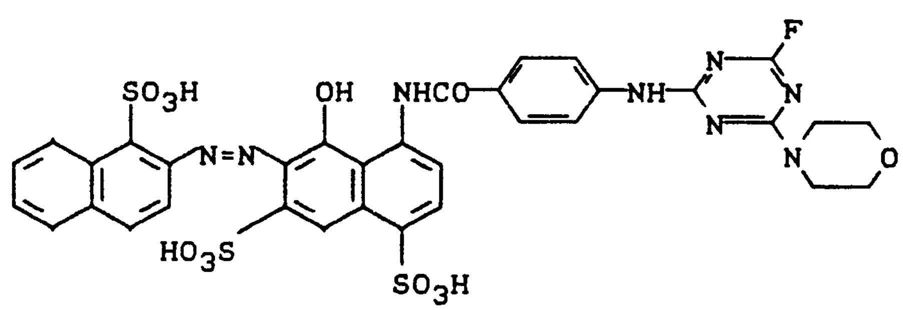

die ebenfalls Baumwolle nach einem für Reaktivfarbstoffe üblichen Applikationsverfahren in klaren blaustichig roten Tönen färben.

Beispiel 11

Ersetzt man in den Beispielen 7-10 das bei der Herstellung der Kupplungskomponente jeweils verwendete Morpholin durch entsprechende Mengen Piperazin, N-Hydroxyethylpiperazin, Piperidin oder Pyrrolidin, so erhält man ebenfalls rote Farbstoffe mit hervorragenden Eigenschaften.

Beispiele 12 - 25

Analog Beispiel 1 lassen sich bei Verwendung der in der folgenden Tabelle aufgeführten Naphthalinsulfonsäuren und Amine weitere interessante Kupplungskomponenten herstellen:

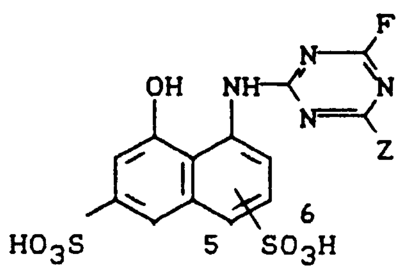

| Nr. | Stellung der Sulfonsäuregruppe | Z |
|---|---|---|
| 12 | 6 | 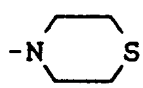 |
| 13 | " | 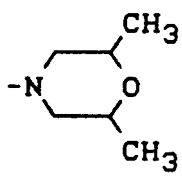 |
| 14 | " | 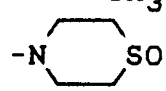 |
| 15 | " | 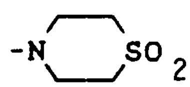 |
| 16 | " | 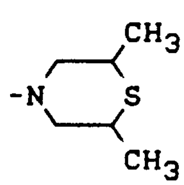 |

| Nr. | Stellung der Sulfonsäuregruppe | Z |
|---|---|---|
| 17 | 6 | 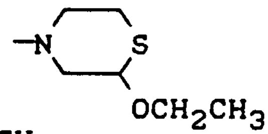 |
| 18 | " | 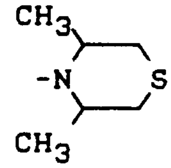 |
| 19 | " | 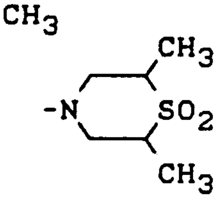 |
| 20 | 5 | 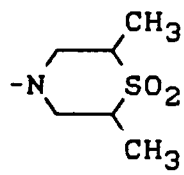 |
| 21 | " | 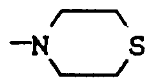 |
| 22 | " | 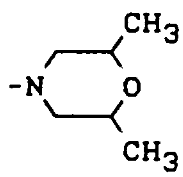 |
| 23 | " | 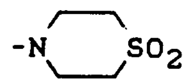 |
| 24 | " | 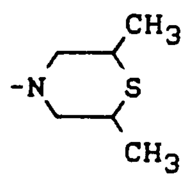 |
| 25 | " | 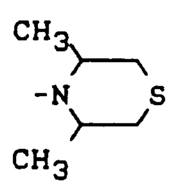 |

Beispiele 26 - 55

Unter Verwendung der in den Beispielen 12 - 25 hergestellten Kupplungskomponenten lassen sich mit den in der folgenden Tabelle aufgeführten Diazokomponenten weitere wichtige Farbstoffe herstellen

D-N=N-K

| Nr. | D | K = Kupplungskomponente aus | Farbton |
|---|---|---|---|
| 26 | 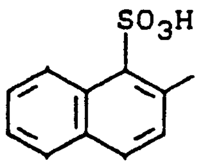 | Beispiel 12 | blaustichig Rot |
| 27 | " | " 13 | " |
| 28 | " | " 14 | " |
| 29 | " | " 15 | " |
| 30 | " | " 16 | " |
| 31 | " | " 17 | " |
| 32 | " | " 18 | " |
| 33 | " | " 19 | " |
| 34 | " | " 20 | Rot |
| 35 | " | " 21 | " |
| 36 | " | " 22 | " |
| 37 | " | " 23 | " |
| 38 | " | " 24 | " |
| 39 | " | " 25 | " |

| Nr. | D | K = Kupplungskomponente aus | Farbton |
|---|---|---|---|
| 40 | 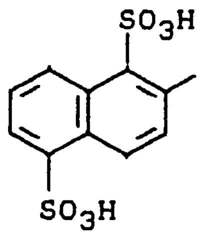 | Beispiel 12 | blaustichig Rot |
| 41 | " | " 13 | " |
| 42 | " | " 15 | " |
| 43 | " | " 21 | Rot |
| 44 | " | " 23 | " |
| 45 | " | " 24 | " |
| 46 | 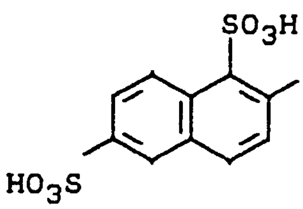 | Beispiel 12 | blaustichig Rot |
| 47 | " | " 13 | " |
| 48 | " | " 15 | blaustichig Rot |
| 49 | " | " 16 | " |
| 50 | " | " 18 | " |
| 51 | " | " 19 | " |
| 52 | " | " 21 | Rot |
| 53 | " | " 22 | " |
| 54 | " | " 23 | " |
| 55 | " | " 24 | " |

## Patentansprüche

**1.** Azofarbstoffe der Formel

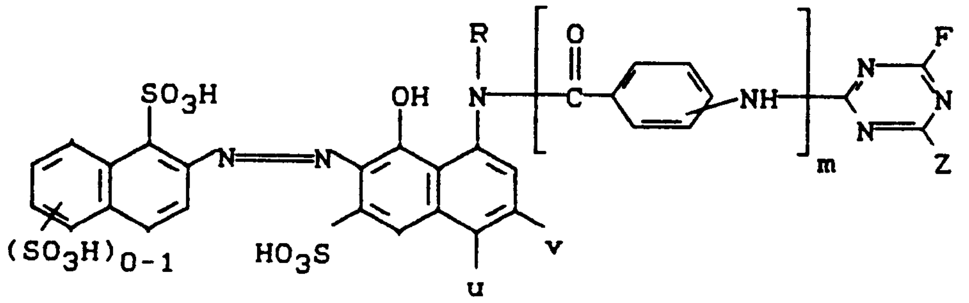

worin

u und v = H oder $SO_3H$ und u ≠ v,

R = H oder $C_1$-$C_4$-Alkyl, wobei die Alkylgruppen gegebenenfalls durch OH, Halogen, $SO_3H$ oder $OSO_3H$ substituiert sein können,

m = 0 oder 1

Z =

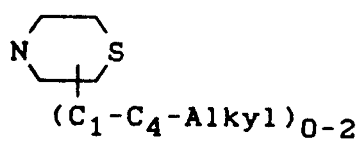

,

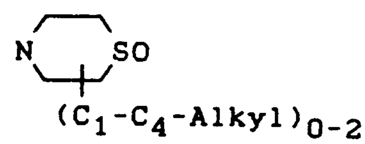

Z =

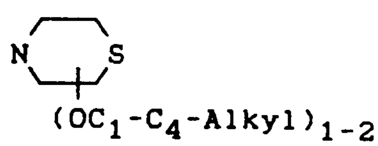

,

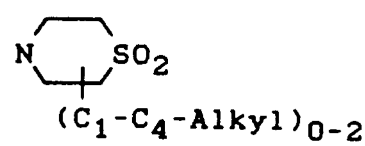

Z =

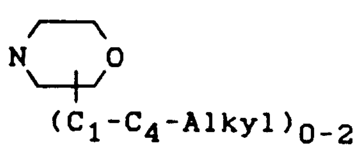

,

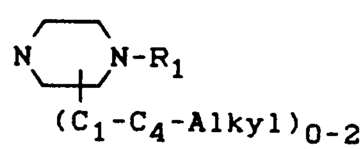

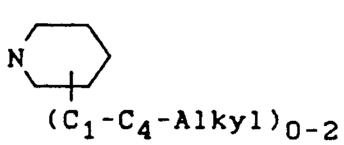

oder

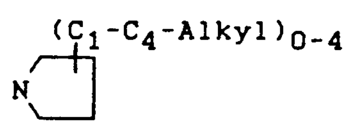

worin

$R_1$ = H oder gegebenenfalls durch wasserlöslich machende Substituenten substituiertes $C_1$-$C_6$-Alkyl.

2. Farbstoffe des Anspruchs 1 mit
Z =

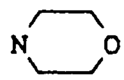 oder 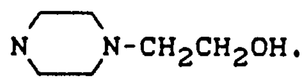

3. Farbstoffe des Anspruchs 1 mit
m = 0.

4. Farbstoff der Formel

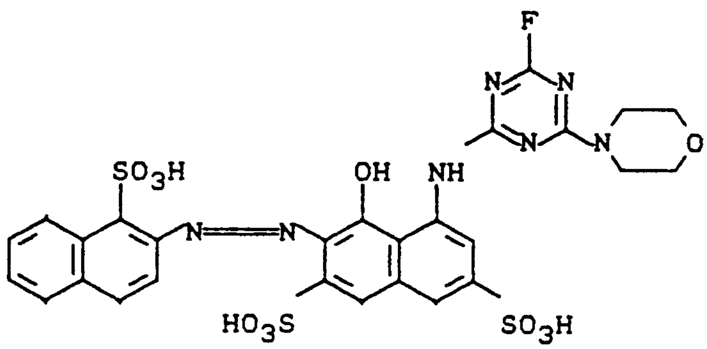

5. Farbstoff der Formel

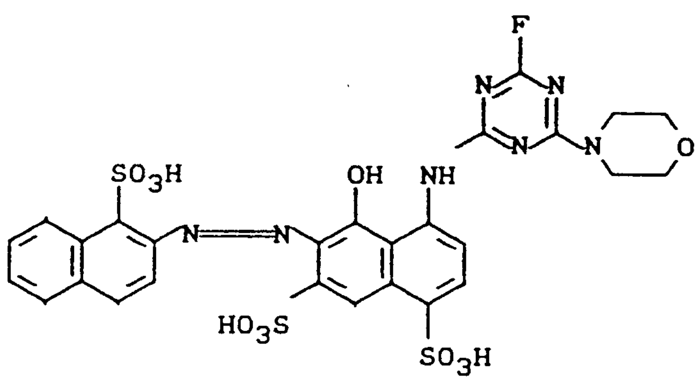

6. Verwendung der Farbstoffe der Ansprüche 1 bis 5 zum Färben und Bedrucken von OH- oder NH-haltigen Materialien.

7. Mit den Farbstoffen der Ansprüche 1 bis 5 gefärbte oder bedruckte OH- oder NH-haltige Materialien.

## Claims

**1.** Azo dyestuffs of the formula

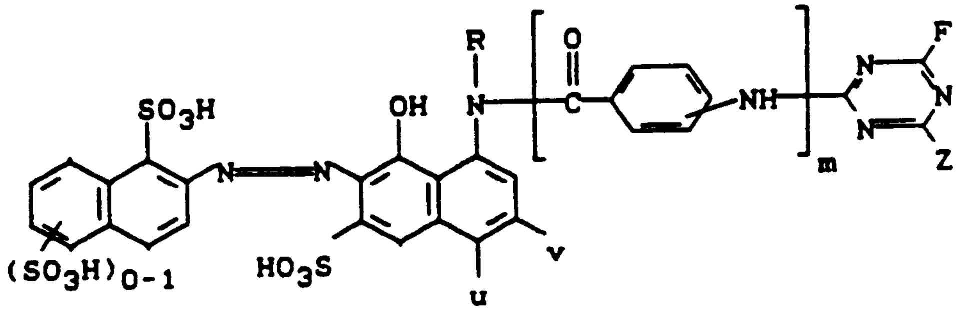

in which

u and v = H or $SO_3H$ and u ≠ v,

R = H or $C_1$-$C_4$-alkyl, it being possible for the alkyl groups to be optionally substituted by OH, halogen, $SO_3H$ or $OSO_3H$,

m = 0 or 1

Z =

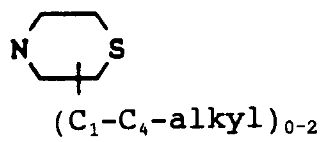

,

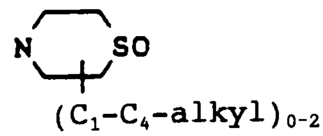

Z =

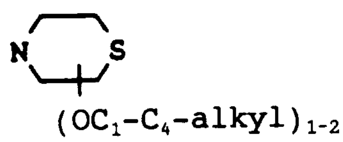

,

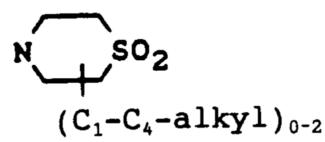

Z =

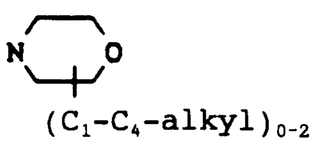

,

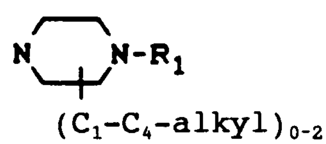

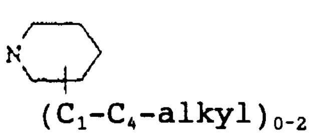

or

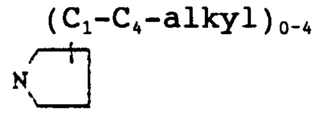

in which

$R_1$ = H or $C_1$-$C_6$-alkyl which is optionally substituted by water-solubilising substituents.

2. Dyestuffs according to Claim 1 where
Z =

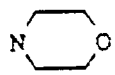

or

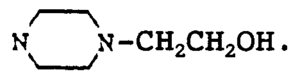

3. Dyestuffs according to Claim 1 where
m = 0.

4. Dyestuff of the formula

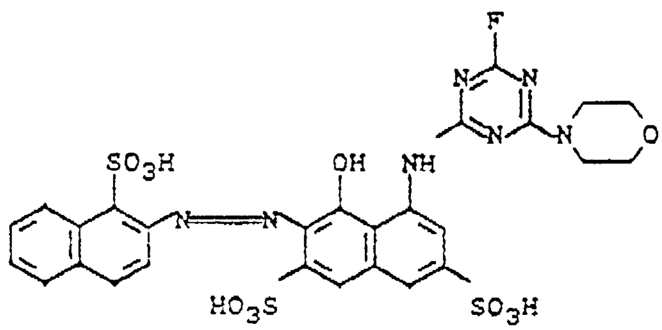

5. Dyestuff of the formula

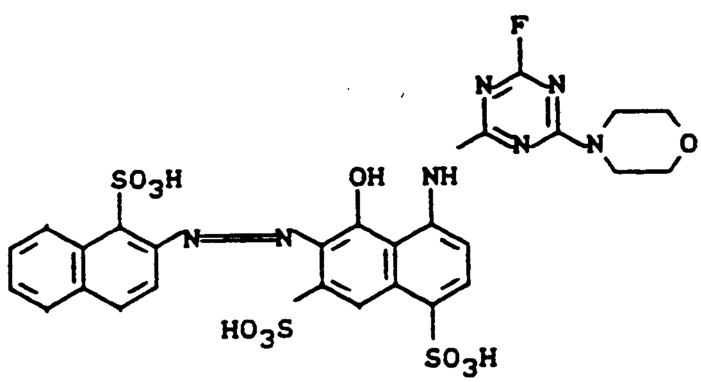

6. Use of the dyestuffs according to Claims 1 to 5 for dyeing and printing OH- or NH-containing materials.

7. OH- or NH-containing materials dyed or printed by the dyestuffs according to Claims 1 to 5.

## Revendications

**1.** Colorant azoïque de formule :

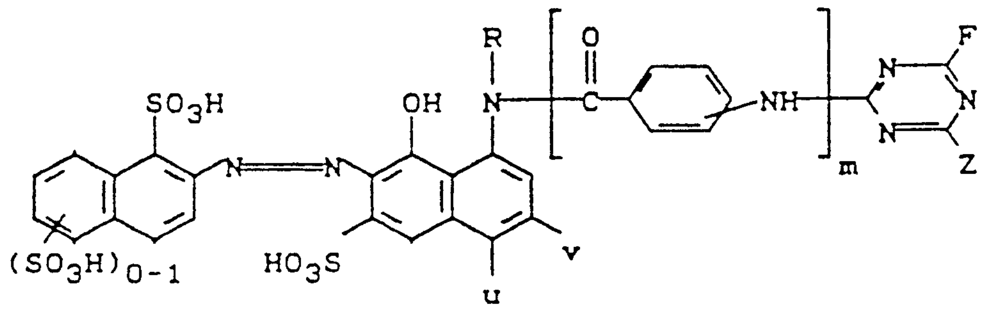

dans laquelle
u et v représentent H ou $SO_3H$ et u est différent de v,
R représente H ou un groupe alkyle en $C_1$-$C_4$ dans lequel le groupe alkyle peut, le cas échéant, être substitué par OH, un atome d'halogène, $SO_3H$ ou $OSO_3H$,
m vaut 0 ou 1
Z =

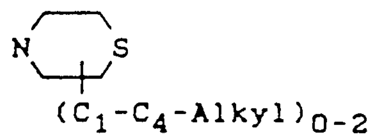
,
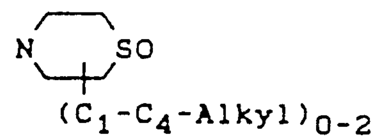

Z =

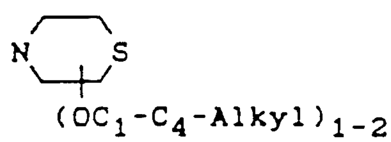
,
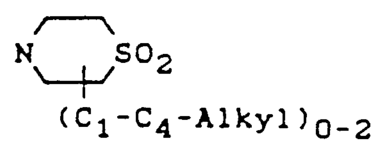

Z =

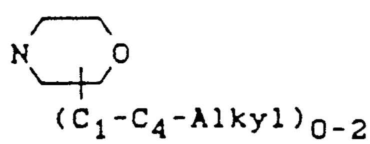
,
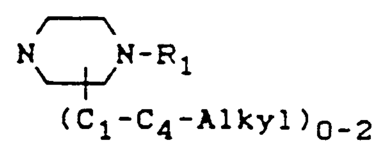

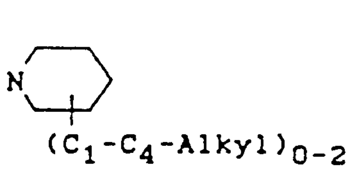
ou
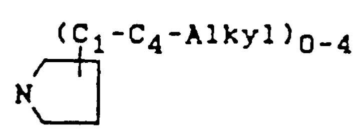

où
$R_1$ représente H ou un groupe alkyle, le cas échéant substitué par un substituant qui le rend soluble dans l'eau.

2. Colorant selon la revendication 1 avec
Z =

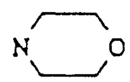

ou

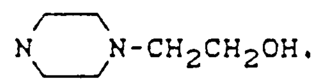

3. Colorant selon la revendication 1 pour lequel m = 0.

4. Colorant de formule :

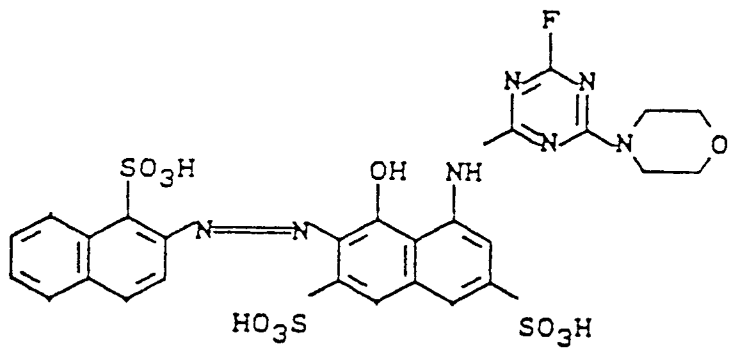

5. Colorant de formule :

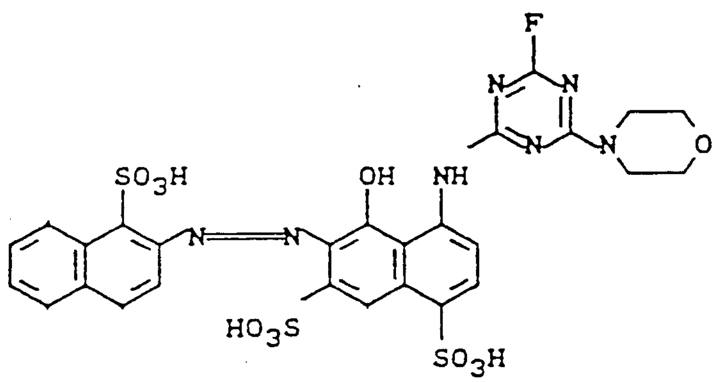

6. Utilisation des colorants des revendications 1 à 5 pour la peinture ou l'impression de matériaux contenant OH ou NH.

7. Matériaux contenant OH ou NH, colorés ou imprimés avec les colorants des revendications 1 à 5.